# FASCICULE DE BREVET EUROPEEN

(11) **EP 3 883 583 B1**
(45) Date de publication et mention de la délivrance du brevet: **28.12.2022**
(21) Numéro de dépôt: 19813432.2
(22) Date de dépôt: 21.11.2019
(51) Int. Cl.: A61K 33/00, A61K 35/08, A61P 17/02, A61K 9/08, A61K 45/06, A61K 8/19, A61Q 19/00

(54) **COMPOSITION CICATRISANTE COMPRENANT UNE EAU ÉLECTROLYSÉE**
HEILENDE ZUSAMMENSETZUNG MIT ELEKTROLYSIERTEM WASSER
HEALING COMPOSITION COMPRISING ELECTROLYZED WATER

(30) Priorité: 21.11.2018 FR 1871652
(43) Date de publication de la demande: 29.09.2021
(73) Titulaire: Waterdiam Group LLC, Miami FL 33136 (US)
(72) Inventeur: PUPUNAT, Laurent, 2800 Delémont (CH); GINTER, Anthony, Miami, 33136 (US)
(74) Mandataire: Gevers & Orès
(86) Numéro de dépôt international: PCT/EP2019/082164
(87) Numéro de publication internationale: WO 2020/104631

(56) Documents cités:
- WO-A1-2014/015443
- WO-A2-2008/029258
- WO-A2-2008/131936
- US-A1- 2006 275 498
- JULIE V. MACPHERSON: "A practical guide to using boron doped diamond in electrochemical research", PHYSICAL CHEMISTRY CHEMICAL PHYSICS, vol. 17, no. 5, 1 janvier 2015 (2015-01-01), pages 2935-2949, XP055246383, ISSN: 1463-9076, DOI: 10.1039/C4CP04022H

## Description

L'invention a pour objet le traitement des plaies et plus particulièrement une composition cicatrisante comprenant une eau électrolysée obtenue à partir d'une eau ayant subi une électrolyse en présence d'électrodes de diamant dopées au bore Le traitement de la cicatrisation de nos jours revêt différentes formes en fonction du degré de gravité de la plaie à traiter.

Pour les blessures bénignes qui saignent un simple pansement fait souvent l'usage. Il en existe de nombreuses sortes sur le marché, avec diverses propriétés. On citera sans être limitatif toutefois la demande WO2O17O27386 qui a pour objet pansement comprenant un support et une matrice élastomérique hydrophobe enrobant ledit support, ladite matrice comprenant un élastomère de type SEPS, SEBS ou SEEPS, constitué d'une association de blocs polystyrène et de blocs polyoléfine, ledit élastomère ayant un poids moléculaire inférieur ou égal à 290 000 Dalton mesuré par chromatographie par perméation de gel, la part totale d'élastomère étant strictement inférieure à 3,0% du poids total de ladite matrice élastomère.

Lorsqu'il s'agit de maladie plus grave ou handicapante telle l'hémophilie, un simple pansement n'est pas suffisant et nécessite l'utilisation de composés chimiques ou biologiques souvent difficiles à extraire ou coûteux afin de soigner les malades. Sans être là encore exhaustif on pourra citer la demande WO20121172O3 qui a pour objet une composition pharmaceutique comprenant un facteur Xa modifié (GDXa) ledit GDXa modifié étant non thrombogène, pouvant se lier au TFPI mais ne pouvant pas se lier aux phospholipides pour la prévention ou le traitement d'un accident hémorragique chez un patient atteint d'hémophilie A ou B avec ou sans inhibiteur.

De nos jours, une maladie plus sournoise que l'hémophilie fait son chemin et provoque des dégâts terribles chez les patients qui en sont atteints, affectant sur le long terme la capacité de l'organisme à cicatriser efficacement. Cette maladie du 21ième siècle c'est le diabète de type II, qui affecte les patients non seulement dans leur vie quotidienne en les obligeant à s'injecter de l'insuline mais aussi à lutter contre les plaies ou coupures au quotidien qui ont du mal à cicatriser jusqu'à conduire parfois à des gangrènes et engager le pronostic vital du malade. Dans la demande WO2001091696 l'invention a pour objet l'utilisation de dérivés de biguanide de formule générale (I) dans laquelle: les groupes R1 et R2 représentent, indépendamment l'un de l'autre, un atome d'hydrogène, un groupe alkyle en C1-C7, un groupe cycloalkyle, un hétérocycle, un groupe alcényle en C2-C7, un groupe aryle, un groupe aralkyle, un groupe aryloxylalkyle ou un groupe hétéroaryle ou R1 et R2 pris ensemble représentent un alkylène en C2-C7 pouvant contenir un ou plusieurs hétéroatomes et le groupe R3 représente une amine primaire, secondaire ou tertiaire ou de leurs sels pharmaceutiquement acceptables pour fabriquer un médicament ayant un effet cicatrisant, ledit médicament étant sous une forme pharmaceutique à usage topique.

Bien qu'il existe un arsenal thérapeutique large afin de soigner les plaies et de les cicatriser efficacement, l'utilisation de produits chimiques ou de facteurs biologiques n'est jamais sans risque, une résistance à une composition pouvant s'installer sur le long terme. Ainsi, il y a un vrai besoin de pouvoir disposer de nouvelles compositions ou composés, permettant efficacement de cicatriser des plaies sans recourir systématiquement à des composés chimiques complexes ou des facteurs biologiques pouvant induire parfois mais pas toujours des rejets ou des effets secondaires limitant ou affectant le processus de cicatrisation.

Le but de l'invention est donc de remédier aux inconvénients précités et de répondre aux besoins précités en proposant une composition comprenant une eau électrolysée pour utilisation en tant que médicament cicatrisant.

Dans un mode de réalisation selon l'invention l'eau électrolysée est obtenue en présence d'au moins une électrode de diamant dopée au bore fixée sur un substrat

L'utilisation de l'eau électrolysée produite à partir d'électrodes de diamant dopées au bore (BDD) dans l'hydratation, le raffermissement et le soin de la peau ainsi que dans le traitement des dermatoses, du psoriasis, des coups de soleil, des escarres et des plaies en général a déjà été divulguée dans WO2014/015443 A1.

Selon la présente invention, la concentration de bore dans l'électrode de diamant est comprise entre 200 ppm (3×10¹⁹ B atomes/cm3) et 2000 ppm (3,52×10²⁰ B atomes/cm3), notamment entre 200 ppm (3×10¹⁹ B atomes/cm3) et 1500 ppm (2×10²⁰ B atomes/cm3)

Le substrat permettant de fixer la dite électrode de diamant est sélectionné parmi le silicium ou le niobium, le tantale ou le tungstène ou un mélange de ceux-ci, préférentiellement le silicium ou le niobium et plus préférentiellement le silicium.

Selon un aspect de l'invention, l'eau électrolysée est obtenue par la mise en œuvre d'un procédé en trois étapes qui débute par l'utilisation d'une eau courante ou de source optionnellement additionnée de chlorure de sodium (NaCl) à la concentration 0,5 à 2g/L suivie d'une électrolyse de ladite eau par un module d'électrolyse comprenant au moins une électrode de diamant dopée au bore fixée sur substrat de silicium dans laquelle la concentration de bore est comprise entre 200 ppm (3×10¹⁹ B atomes/cm³) et 2000 ppm (3,52×10²⁰ B atomes/cm³), notamment entre 200 ppm (3×10¹⁹ B atomes/cm³) et 1500 ppm (2×10²⁰ B atomes/cm³), le dit module soumettant l'eau à une quantité de courant (densité de courant) lors du processus d'électrolyse comprise entre 15 et 500 mAh/L d'eau, plus préférentiellement 40 à 250 mAh/L d'eau, encore plus préférentiellement 50 à 200 mAh/L, la durée de l'électrolyse étant comprise entre 15 et 30 minutes.

Par ailleurs, l'eau utilisée dans la composition selon l'invention est une naturelle ou purifiée sans qu'un ajout d'au moins un additif ne soit nécessaire.

Dans un autre mode de réalisation de l'invention la composition selon l'invention peut comprendre également un agent de cicatrisation naturel ou de synthèse.

L'agent de cicatrisation naturel ou de synthèse est toujours présent à une concentration inférieure à celle d'une composition équivalente comprenant une eau classique ou distillée non électrolysée.

La concentration en agent cicatrisation naturel ou de synthèse est inférieure de 25 à 75% à celle d'une composition équivalente comprenant une eau classique ou distillée non électrolysée.

De préférence l'agent de cicatrisation naturel ou de synthèse est sélectionné parmi la metformine, le cuivre ou ses dérivés, la papavérine, le bendazole, un extrait de calendula, d'aloé vera, des huiles essentielles cicatrisantes, le tétrapeptide Acétyl-Ser-Asp-Lys-Pro AcSDKP, le zinc ou ses dérivés, la provitamine B5, le sucralfate, le resveratrol, la lanoline, la vitamine A, l'allantoïne, l'acide hyaluronique, le tocophérol ou ses dérivés, la souci des jardins, l'huile d'amande douce ou encore de jojoba, de calophylle ou de millepertuis ou un mélange de ceux-ci.

La composition selon l'invention comprend également un ou plusieurs excipients et/ou des émulsifiants naturels ou de synthèse sélectionnés parmi la vaseline, la glycérine, la paraffine, le cetearyl glucose, la cire d'abeille ou de riz, la lécithine de soja, des esters de sucre, le glyceryl stearate, des dérivés d'huile d'olive ou un mélange de ceux-ci.

Dans un mode de réalisation, la composition selon l'invention comprend de 60 à 95 parties en poids d'eau électrolysée, de 0 à 10 parties en poids d'agent cicatrisant de synthèse ou naturel et de 1 à 10 parties en poids d'un excipient et/ou émulsifiant.

Dans un autre mode de réalisation, la composition selon l'invention comprend de 60 à 95 parties en poids d'un excipient et/ou émulsifiants, de 0 à 10 parties en poids d'agent cicatrisant de synthèse ou naturel et de 10 à 30 parties en poids d'eau électrolysée .

La composition selon l'invention peut se présenter également sous forme de crème, de gel ou d'émulsion.

L'invention a également pour objet l'utilisation d'une composition selon l'invention pour le traitement des affections de la peau générant des plaies ou des défauts de cicatrisation, tel les escarres, des érysipèles, les blessures ouvertes, les ulcères variqueux.

L'eau électrolysée formant la base de la composition de l'invention peut avantageusement être obtenue par un procédé utilisant un un module d'électrolyse comprenant au moins une electrode de diamant dopée au bore fixée sur substrat de silicium dans laquelle la concentration de bore est comprise entre 200 ppm (3x10¹⁹ B atoms/cm³) et 2000 ppm (3,52×10²⁰ B atomes/cm³), notamment entre 200 ppm (3×10¹⁹ B atoms/cm³) et 1500 ppm (2×10²⁰ B atoms/cm³), le dit module soumettant l'eau à une quantité du courant lors du processus d'électrolyse comprise entre 15 et 500 mAh/L d'eau, plus préférentiellement 40 à 250 mAh/L d'eau, encore plus préférentiellement 50 à 200 mAh/L.

La durée d'électrolyse peut se faire sur une durée plus ou moins longue, généralement inférieure ou égale à 60 minutes, notamment comprise entre 15 et 60 minutes, en particulier entre 15 et 30 minutes. Typiquement, une durée entre 5 et 30 minutes est une durée raisonnable. L'électrolyse peut s'effectuer par cycles, entre 4 et 12 cycles de traitement de l'eau par 24 heures.

L'invention a également pour objet enfin un applicatif de type adhésif, pansement, un patch ou encore un masque pour la peau comprenant ou étant obtenu à partir d'une composition selon l'invention.

La composition de l'invention permettra notamment de produire un masque pouvant être appliqué sur la peau ou le visage, ledit masque étant obtenu à partir d'une poudre telle une argile, un exfoliant, un extrait de plante en poudre ou une argile contenant du charbon actif et additionnée d'eau électrolysée obtenue selon le procédé décrit ci-dessus

Les dessins annexés illustrent l'invention :
La Figure 1 représente l'action d'une composition selon l'invention comportant une eau électrolysée obtenue par électrolyse d'eau par électrodes A de diamant dopées au bore (1200 ppm de bore) sur substrat de silicium sur le processus de cicatrisation d'une plaie.
La Figure 2] représente un test comparatif entre un traitement avec une eau classique (contrôle) et une eau électrolysée dans une composition selon l'invention sur le processus de cicatrisation.
La Figure 3 représente l'action d'une eau électrolysée obtenue par électrolyse d'eau par électrodes B de diamant dopées au bore (2500 ppm de bore) sur substrat de silicium sur le processus de cicatrisation d'une plaie.

La présente invention va être décrite de façon plus détaillée et à l'aide d'un ou plusieurs exemples qui ne limitent nullement l'invention.

La cicatrisation des plaies est un mécanisme complexe, impliquant de nombreuses protéines et mécanismes cellulaires de reconstruction de la plaie. On peut décomposer le processus en trois phases ; une phase vasculaire et inflammatoire, précoce, appelée aussi phase de détersion ou phase exsudative pour la détersion d'une plaie, une seconde phase dite de bourgeonnement qui correspond à la phase proliférative avec développement du tissu de granulation, le tissu permettant de combler la perte de substance par un nouveau tissu grâce à la néo-angiogenèse et la prolifération cellulaire. La phase de bourgeonnement se poursuit jusqu'à l'épithélialisation. Il s'en suit une phase plus longue dite de remodelage de la cicatrice.

Plusieurs solutions existent sur le marché pouvant être utilisées selon la gravité des plaies à traiter. Mais on ne traite pas de la même façon une simple égratignure et un ulcère variqueux. Il est donc utile de pouvoir disposer de nouveaux traitements par rapport à ceux conventionnellement utilisés principalement basés sur l'utilisation de composés chimiques naturels ou de synthèse ou encore de pansements.

L'avantage de la présente invention est que l'utilisation de médicaments s'en trouve réduite voire éliminée pour des cicatrisations superficielles ou plus complexes et est simple à mettre en œuvre du fait de la simplicité de la composition. De par sa nature, on peut également « réactiver » la dite composition. Une telle réactivation s'entend de la capacité à appliquer de manière répétée un traitement d'électrolyse de la composition qui permet en quelque sorte de régénérer l'eau produit utile présent dans la composition. De plus, avantage non négligeable, le coût de la composition du fait de sa matière première est inférieur à celui des compositions conventionnelles. L'accessibilité aisée du produit principal compris dans la composition selon l'invention est aussi un de ces avantages.

La composition selon l'invention utile dans le traitement de la cicatrisation ou de désordres associés est principalement composée par une eau électrolysée obtenue en utilisant une station portable ou fixe d'électrolyse sans membrane comprenant au moins une électrode de diamant dopée au bore fixée sur un substrat pouvant être en silicium, en niobium, en tantale, en tungstène ou un mélange de ceux-ci.

Selon l'invention, l'utilisation de substrats de silicium ou de niobium est recommandée, le silicium étant le substrat préférentiel.

Sans être lié par la théorie, les électrodes de diamant dopées au bore (dites BDD) présentes sur un substrat silicium permettent d'atteindre des potentiels d'électrolyse élevés, supérieurs aux électrodes de platine conventionnellement utilisées pour l'électrolyse de l'eau. Ainsi ces électrodes BDD permettent de générer des modifications structurelles qui vont conférer à l'eau un potentiel thérapeutique et plus particulièrement sur les processus de cicatrisation.

Dans un mode préféré de l'invention la concentration de bore présente dans les électrodes est comprise entre 200 ppm (3×10¹⁹ B atomes/cm³) et 1500 ppm (2×10²⁰ B atomes/cm³) constituant un optimum afin d'obtenir une eau électrolysée de qualité.

Un plus faible taux de bore ne permet pas d'obtenir une eau électrolysée de qualité et un plus fort taux de bore conduisant à une réduction des performances des électrodes ainsi qu'une dégradation de la durée de vie des électrodes.

L'eau électrolysée base de la composition inventive est obtenue par un procédé utilisant un module d'électrolyse comprenant au moins une électrode de diamant dopée au bore fixée sur substrat de silicium tel que précédemment évoquée, le dit module soumettant l'eau une quantité du courant appliquée à l'eau lors du processus d'électrolyse est comprise entre 15 et 500 mAh/L d'eau, plus préférentiellement 40 à 250 mAh/L d'eau, encore plus préférentiellement 50 à 200 mAh/L.

La durée d'électrolyse peut se faire sur une durée plus ou moins longue, entre 5 et 30 minutes. L'électrolyse peut s'effectuer par cycles, entre 4 et 12 cycles de traitement de l'eau par 24 heures peut être envisagé.

Sans être lié par la théorie, l'eau obtenue par le procédé décrit précédemment n'est pas caractérisable en tant que telle et c'est cette eau obtenue par le procédé qui présente les propriétés cicatrisante selon l'invention. Pour preuve l'utilisation d'une eau obtenue par la mise en œuvre du procédé décrit précédemment obtenue en utilisant non pas une électrode de diamant dopée au bore mais une électrode de platine classique ne permet pas d'obtenir une composition selon l'invention et ses effets avantageux.

Dans la composition selon l'invention, l'eau électrolysée peut être utilisée juste avant de faire ladite composition et, ce qui présente un avantage, peut être électrolysée à nouveau à tout moment, ce qui permet de maintenir le potentiel cicatrisant de ladite composition très longtemps et permet en quelque sorte de régénérer ladite composition par un procédé relativement simple.

L'eau utilisée peut être une eau courante, purifiée ou thermale du commerce connue pour ses propriétés ou vertus thérapeutiques. Nul besoin que l'eau électrolysée soit une eau distillée. En outre, l'invention ne nécessite pas d'ajout d'au moins un additif tel que ceux utilisés lors de procédés de préparation d'eau électrolysée que l'on utilise pour nettoyer les piscines ou les spas à savoir du chlore à de fortes concentrations ou encore de l'oxygène actif.

De plus, un des avantages de l'utilisation de l'eau électrolysée par une électrode de type BDD dans une composition selon l'invention est qu'en présence d'un agent cicatrisant naturel ou de synthèse connu, la présence de celle-ci permet d'utiliser moins d'agent cicatrisant naturel ou de synthèse que si on utilise une composition seule le contenant en présence d'une eau naturelle ou courante non électrolysée ou distillée.

Ainsi, dans des compositions selon l'invention pouvant comprendre de tels agents, l'agent de cicatrisation naturel ou de synthèse sera toujours présent à une concentration inférieure à celle d'une composition équivalente comprenant une eau classique ou distillée non électrolysée. Ce qui présente l'avantage de réduire la dépendance ou l'inefficacité d'un produit sur le long terme. Car pour certaines maladies la cicatrisation est un problème récurrent, l'exposition prolongée ou régulière peut provoquer une accoutumance ou une résistance par l'organisme vis-à-vis de ces agents.

La concentration en agent de cicatrisation naturel ou de synthèse dans la composition selon l'invention sera inférieure de 25 à 75% à celle d'une composition équivalente vendue dans le commerce ne comprenant pas d'eau électrolysée mais une eau classique ou distillée.

Telle que mentionnée précédemment, la composition selon l'invention peut comprendre également un agent de cicatrisation naturel ou de synthèse. Il est à noter que celui-ci ne s'entend pas comme un additif dans le cadre de la présente invention, les additifs tels que mentionnés précédemment s'entendent comme des additifs de type organique ou inorganique sous forme de sels servant à purifier l'eau.

L'agent de cicatrisation naturel ou de synthèse s'entend dans le cadre de la présente invention comme des produits ayant été reconnus dans des brevets ou la littérature scientifique, connus de l'homme du métier dans le domaine des produits cicatrisants comme des produits susceptibles d'être utilisés comme agents cicatrisants ou dans des maladies apparentées comme des affections de la peau générant des plaies ou des défauts de cicatrisation, tel les escarres, des érysipèles, les blessures ouvertes, les ulcères variqueux.

Sans toutefois être exhaustif, l'agent de cicatrisation naturel ou de synthèse peut être sélectionné parmi la metformine, du cuivre ou ses dérivés, la papavérine, le bendazole, un extrait de calendula, d'aloé vera, des huiles essentielles cicatrisantes, AcSDKP, le zinc ou ses dérivés, la provitamine B5, le sucralfate, le resveratrol, la lanoline, la vitamine A, l'allantoïne, l'acide hyaluronique, le tocophérol ou ses dérivés, la souci des jardins, l'huile d'amande douce ou encore de jojoba, de calophylle ou de millepertuis ou un mélange de ceux-ci.

La composition selon l'invention peut également comprendre des excipients et/ou des émulsifiants naturels ou de synthèse, et ce conformément aux principes galéniques connus de l'homme du métier visant à mettre la composition sous forme de crème. Sans être exhaustif, ceux-ci peuvent être sélectionnés parmi la vaseline, la glycérine, la paraffine, le cetearyl glucose, la Cire d'abeille ou de riz, la lécithine de soja, des esters de sucre, le glyceryl stearate, des dérivés d'huile plus particulièrement d'huile d'olive ou un mélange de ceux-ci.

En fonction du type de crème que l'on veut obtenir et en fonction de son pouvoir pénétrant recherché, on peut mettre dans la crème un pourcentage plus ou moins élevé d'eau électrolysée par rapport aux émulsifiants ou inversement. Dans le cas d'une émulsion dite eau dans huile aussi nommée E/H, la quantité d'huile est supérieure à la quantité d'eau. L'émulsion ainsi obtenue est très nourrissante, hydratante et protectrice car elle crée un film lipidique sur la peau. On l'utilise idéalement pour les peaux sèches ou les crèmes de nuit.

Dans le cas d'une émulsion dite huile dans eau aussi nommé H/E, la quantité d'eau est supérieure à la quantité d'huile. Ce type d'émulsion est nourrissant et hydratant. On l'utilise idéalement pour réaliser des crèmes de jour, des laits corporels, elle peut être aussi intégrée dans des patchs ou des adhésifs de type pansement.

Une autre forme galénique potentielle pour la composition selon l'invention peut être un gel aqueux aussi appelé hydrogel. Un hydrogel est un gel dans lequel l'agent gonflant est l'eau. La matrice d'un hydrogel est généralement un réseau de polymères qui sont insolubles dans l'eau, mais sont capables de gonfler substantiellement en présence d'une grande quantité d'eau ou de solutions aqueuses.

Ainsi plusieurs formulations ou mises en forme de la composition selon l'invention peuvent être possibles selon les besoins du patient à traiter et l'importance du processus de cicatrisation à mettre en œuvre. Elle peut se présenter sous forme de crème, de gel ou d'émulsion ou encore sous forme d'un adhésif de type pansement ou un patch pour la peau.

En fonction du type d'émulsion recherché ou de composition recherchée la composition selon l'invention dans le cas d'une composition dite H/E comprend de 60 à 95 parties en poids d'eau électrolysée de 0 à 10 parties en poids d'agent cicatrisant de synthèse ou naturel et de 1 à 10 parties en poids d'un excipient et/ou émulsifiants.

Dans le cas d'une composition dite E/H, ladite composition comprend de 60 à 95 parties en poids d'un excipient et/ou émulsifiants, de 0 à 10 parties en poids d'agent cicatrisant de synthèse ou naturel et de 10 à 30 parties en poids d'eau électrolysée.

Les différentes formulations galéniques possibles de la composition selon l'invention sont faites pour utilisation en tant que médicament cicatrisant ou le traitement des affections de la peau de la peau générant des plaies ou des défauts de cicatrisation, tel les escarres, des érysipèles, les blessures ouvertes ou encore les ulcères variqueux.

### Exemples

### Exemple 1 : Etude des effets de la concentration en bore de l'électrode sur l'efficacité de la cicatrisation d'une plaie

La vitesse de cicatrisation de fibroblastes en fonction de la teneur en bore des électrodes utilisées pour électrolyser l'eau de culture a été étudiée.

Les électrodes dopées au bore (ci-après désignées électrodes BDD/Si) **A** et **B** mises en œuvre dans cette expérience présentent les caractéristiques suivantes :
- Electrodes BDD/Si : film de diamant dopé au bore sur substrat silicium :
- Substrat : silicium monocristallin (100), résistivité 100 mohm.cm
- film BDD : polycristallin, épaisseur ∼2-3µm, dopage 1200 ppm de bore (Electrode **A)** ou 2500 ppm de bore (Electrode **B),**

Les électrodes **A** et **B** ont été fabriquées selon le même protocole de croissance de film diamant en machine HF-CVD (Hot Filament Chemical Vapor Déposition. Elles sont identiques en tout point et ne diffèrent que par leur teneur respective en bore.

### a) Le protocole de mise en œuvre des électrodes sur l'eau de culture est le suivant :

Un réservoir de 2,5 L contient de l'eau de ville à 15 °C. Cette eau est pompée à un débit fixe de 200 L/h à travers un module d'électrolyse puis renvoyée dans le réservoir donc selon un circuit fermé. Le module d'électrolyse met en œuvre de 2 électrodes espacées de 1 mm et de 70cm² de surface active. Le courant d'électrolyse est 2A pendant des périodes de travail t = 0 - 1 - 2 - 5 - 10 - 20 - 30 - 40 min de manière à atteindre des charges d'électrolyse de 0 à 533 mAh/L. L'eau du réservoir est maintenue à une température constante de 20°C lors de l'essai.

L'eau électrolysée est échantillonnée en sortie de module d'électrolyse puis tout de suite filtrée stérile (filtres à membrane poreuse de 0,2 µm) et ajoutée au milieu de culture des fibroblastes avec une dilution de 1:4 (= concentration de 25 %).

### b) Le protocole de culture est le suivant:

Fibroblastes : L-929 (fibroblastes de souris; ACC 173; DSMZ); passage interne P52-53; recommandé selon EN ISO 10993-5: 2009). Les cellules sont incubées et cultivées en masse dans un incubateur à 37 °C avec un environnement fermé maîtrisé contenant 5 % de CO2 et 95 % air. Le milieu de culture est le RPMI 1640 avec 10 % de sérum physiologique de bovin, 100 Unit/mL de pénicilline et 100 µg/mL de streptomycine.

### c) Le protocole d'étude de la régénération/cicatrisation des cellules est le suivant:

Utilisation d'inserts de culture en silicone (ibidi GmbH, München). Lorsque cet insert est disposé dans un milieu de culture, il constitue 2 réservoirs de culture séparés par une paroi de 500 µm d'épaisseur. Les cellules sont cultivées dans les deux réservoirs puis l'insert en silicone est retiré. Cela permet d'obtenir deux patches de culture parfaitement définis et espacés précisément de 500 µm.

Pour les expériences, des cellules type L-929 ont été obtenues à partir de 80 à 90 % en masse de cultures en suspension d'une densité de 500,000 cellules/ml. 100 µL de suspension sont introduits dans chaque réservoir d'insert de culture. Les cellules sont cultivées pendant 24 heures afin d'obtenir des populations homogènes dans chacun des deux réservoirs de chaque insert. Puis, l'insert est délicatement retiré, laissant apparaître un espace libre de 500 µm séparant les deux milieux de cultures. L'eau électrolysée est injectée à hauteur de 500 µL pour 1,500 µL de milieu de culture frais (dilution 1:4). Les milieux de culture sont cultivés à nouveau pendant 24 heures. Puis, des couches de cellules ont été fixées par un traitement au méthanol pendant 2 min et colorées au moyen d'une solution Coomassie-Giemsa selon Romanowsky.

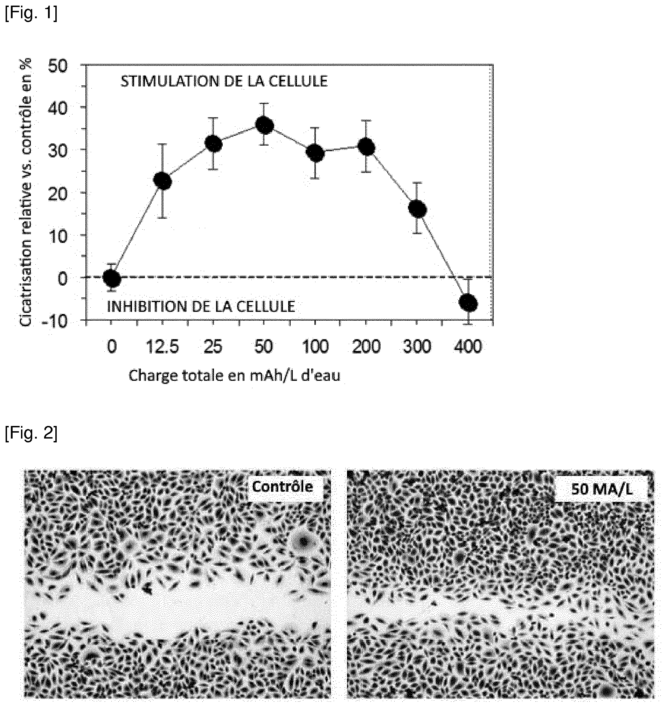

L'espace de séparation est photographié via un écran de 27" afin d'observer en 5 points le long de l'espace, la vitesse de rapprochement des deux milieux, jusqu'à la jonction (Figure 2).

### d) Résultats

Les résultats avec l'eau électrolysée avec l'électrode BDD **A** (dopage bore 1200 ppm B) montrent une accélération significative de la vitesse de cicatrisation par rapport à l'eau stérile sans électrolyse, jusqu'à plus de 30% à partir de 25 mAh/L jusqu'à 200 mAh/L puis une diminution (Figure 1).

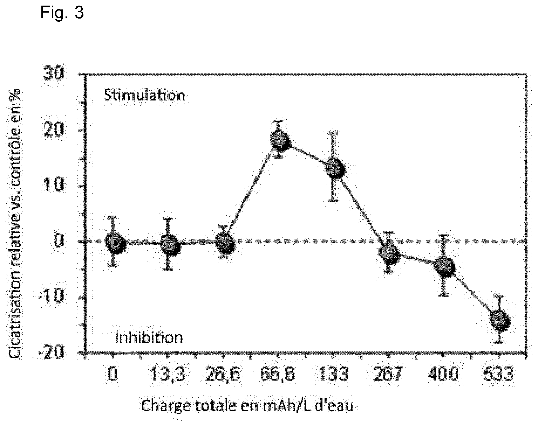

Avec l'électrode BDD **B** (env. 2500 ppm B), on observe une accélération de la cicatrisation mais bien inférieure à celle obtenue avec les électrodes **A** (Figure 3).

### Exemple 2 = Teneur en bore de l'eau électrolysée

Le protocole d'électrolyse d'eau de robinet est le suivant :
L'eau mise en œuvre est une eau de ville.

Le protocole d'électrolyse est le suivant :
L'eau de ville est pompée à un débit de 90 L/h à travers un module d'électrolyse équipé de 2 électrodes BDD **A** telles que définies à l'exemple 1 (1200 ppm B) espacées de 1mm, ayant 12.5cm² de surface active.

Le courant appliqué est de 2.4A. L'échantillon d'eau est collecté directement à la sortie du module d'électrolyse (boucle ouverte, fonctionnement dit à eau perdue).

Les résultats sont rapportés dans les tableaux ci-dessous :
Echantillon 1 : eau brute de ville
Echantillons 2 et 3 : eau électrolysée avec des électrodes A (1200 ppm de bore)

| | Echantillon | Bore | Si |
|---|---|---|---|
| 1 | brute ville | 31,5 | 4,7 |
| 2 | mini cellule débit faible 2.4A | 34 | 4,54 |
| 3 | mini cellule débit fort 2.2A | 34,2 | 4,54 |
| | | µg/L | mg/L |
| | relargage | 2,60 | µg B/L |
| | débit moyen | 90 | L/h |
| courant moyen | 2,3 | A | |
| | 101,74 | µg B/Ah | |

L'électrolyse de l'eau avec les électrodes A provoque une faible mais notable augmentation de la concentration en bore dans l'eau électrolysée vis-à-vis de l'eau brute : environ +101,74 microgramme de bore/Ah de charge électrique appliquée. Les mesures de bore dans l'eau sont réalisées par Spectrométrie de masse à plasma induit (ICP-MS).

## Revendications

1. Composition comprenant une eau électrolysée pour utilisation en tant que médicament cicatrisant, dans laquelle l'eau électrolysée est obtenue par électrolyse au moyen d'au moins une électrode de diamant dopée au bore fixée sur un substrat, dans laquelle la concentration de bore est comprise entre 200 (3×10¹⁹ B atomes/cm³) et 2000 ppm (3,52×10²⁰ B atomes/cm³), notamment entre 200 ppm (3×10¹⁹ B atomes/cm³) et 1500 ppm (2×10²⁰ B atomes/cm³)

2. Composition pour utilisation selon la revendication 1, dans laquelle l'eau est une eau naturelle ou purifiée sans ajout d'au moins un additif.

3. Composition pour utilisation selon l'une quelconque des revendications précédentes, laquelle composition comprend également un agent de cicatrisation naturel ou de synthèse.

4. Composition pour utilisation selon la revendication 3, dans laquelle l'agent de cicatrisation naturel ou de synthèse est sélectionné parmi la metformine, du cuivre ou ses dérivés, la papavérine, le bendazole, un extrait de calendula, d'aloé vera, des huiles essentielles cicatrisantes, le tétrapeptide Acétyl-Ser-Asp-Lys-Pro (AcSDKP), le zinc ou ses dérivés, la provitamine B5, le sucralfate, le resveratrol, la lanoline, la vitamine A, l'allantoïne, l'acide hyaluronique, le tocophérol ou ses dérivés, la souci des jardins, l'huile d'amande douce, de jojoba ou de millepertuis ou un mélange de ceux-ci.

5. Composition pour utilisation selon l'une quelconque des revendications précédentes, dans laquelle sont présents également des excipients et/ou des émulsifiants naturels ou de synthèse sélectionné parmi la vaseline, la glycérine, la paraffine, le cetearyl glucose, la cire d'abeille ou de riz, la lécithine de soja, des esters de sucre, le glyceryl stearate, des dérivés d'huile d'olive ou un mélange de ceux-ci.

6. Composition pour utilisation selon l'une quelconque des revendications précédentes, dans laquelle la composition comprend de 60 à 95 parties en poids d'eau électrolysée de 0 à 10 parties en poids d'agent cicatrisant de synthèse ou naturel et de 1 à 10 parties en poids d'un excipient et/ou émulsifiants.

7. Composition pour utilisation selon l'une quelconque des revendications 1 à 5 dans laquelle, la composition comprend de 60 à 95 parties en poids d'un excipient et/ou émulsifiants, de 0 à 10 parties en poids d'agent cicatrisant de synthèse ou naturel et de 10 à 30 parties en poids d'eau électrolysée .

8. Composition selon l'une quelconque des revendications précédentes pour utilisation en tant que médicament cicatrisant dans le traitement des affections de la peau générant des plaies ou des défauts de cicatrisation, tel les escarres, les érysipèles, les blessures ouvertes, les ulcères variqueux.

9. Masque d'application cutanée comportant une composition selon l'une des revendications 1 à 8.

## Patentansprüche

1. Zusammensetzung, umfassend elektrolysiertes Wasser zur Verwendung als Wundheilungsmedikament, wobei das elektrolysierte Wasser durch Elektrolyse mittels mindestens einer auf einem Substrat befestigten bordotierten Diamantelektrode erhalten wird, wobei die Borkonzentration zwischen 200 (3×10¹⁹ B Atome/cm³) und 2000 ppm (3,52×10²⁰ B Atome/cm³) liegt, insbesondere zwischen 200 ppm (3×10¹⁹ B Atome/cm³) und 1500 ppm (2×10²⁰ B Atome/cm³)

2. Zusammensetzung zur Verwendung nach Anspruch 1, wobei das Wasser ein natürliches oder gereinigtes Wasser ohne Zusatz von mindestens einem Zusatzstoff ist.

3. Zusammensetzung zur Verwendung nach einem der vorstehenden Ansprüche, wobei die Zusammensetzung ebenfalls einen natürlichen oder synthetischen Wundheilungswirkstoff umfasst.

4. Zusammensetzung zur Verwendung nach Anspruch 3, wobei der natürliche oder synthetische Wundheilungswirkstoff ausgewählt ist aus Metformin, Kupfer oder seinen Derivaten, Papaverin, Bendazol, einem Extrakt aus Calendula, Aloe Vera, ätherischen Wundheilungsölen, dem Tetrapeptid Acetyl-Ser-Asp-Lys-Pro (AcSDKP), Zink oder seinen Derivaten, Provitamin B5, Sucralfat, Resveratrol, Lanolin, Vitamin A, Allantoin, Hyaluronsäure, Tocopherol oder seinen Derivaten, Ringelblume, Süßmandel-, Jojoba- oder Johanniskrautöl oder einer Mischung davon.

5. Zusammensetzung zur Verwendung nach einem der vorstehenden Ansprüche, wobei ebenfalls natürliche oder synthetische Hilfsstoffe und/oder Emulgatoren vorliegen, die ausgewählt sind aus Vaseline, Glycerin, Paraffin, Cetearylglucose, Bienen- oder Reiswachs, Sojalecithin, Zuckerestern, Glycerylstearat, Olivenölderivaten oder einer Mischung davon.

6. Zusammensetzung zur Verwendung nach einem der vorstehenden Ansprüche, wobei die Zusammensetzung 60 bis 95 Gewichtsteile elektrolysierten Wassers, 0 bis 10 Gewichtsteile synthetischen oder natürlichen Wundheilungswirkstoffs und 1 bis 10 Gewichtsteile eines Hilfsstoffs und/oder Emulgatoren umfasst.

7. Zusammensetzung zur Verwendung nach einem der Ansprüche 1 bis 5, wobei die Zusammensetzung 60 bis 95 Gewichtsteile eines Hilfsstoffs und/oder Emulgatoren, 0 bis 10 Gewichtsteile synthetischen oder natürlichen Wundheilungswirkstoffs und 10 bis 30 Gewichtsteile elektrolysierten Wassers umfasst.

8. Zusammensetzung nach einem der vorstehenden Ansprüche zur Verwendung als Wundheilungsmedikament bei der Behandlung von Erkrankungen der Haut, die Wunden oder Wundheilungsstörungen verursachen, wie z. B. Dekubitus, Erysipel, offene Wunden, Krampfadergeschwüre.

9. Maske zur Anwendung auf der Haut, die eine Zusammensetzung nach einem der Ansprüche 1 bis 8 aufweist.

## Claims

1. A composition comprising electrolyzed water for use as a healing medicament, wherein the electrolyzed water is obtained by electrolysis using at least one boron-doped diamond electrode attached to a substrate, wherein the concentration of boron is between 200 (3×10¹⁹ B atoms/cm³) and 2000 ppm (3.52×10²⁰ B atoms/cm³), in particular between 200 ppm (3×10¹⁹ B atoms/cm³) and 1500 ppm (2×10²⁰ B atoms/cm³)

2. The composition for use according to claim 1, wherein the water is natural or purified water without addition of at least one additive.

3. The composition for use according to any one of the preceding claims, which composition also comprises a natural or synthetic healing agent.

4. The composition for use according to claim 3, wherein the natural or synthetic healing agent is selected from metformin, copper or its derivatives, papaverine, bendazole, an extract of calendula, aloe vera, healing essential oils, Acetyl-Ser-Asp-Lys-Pro (AcSDKP) tetrapeptide, zinc or its derivatives, provitamin B5, sucralfate, resveratrol, lanolin, vitamin A, allantoin, hyaluronic acid, tocopherol or its derivatives, marigold, sweet almond oil, jojoba or St. John's wort oil or a mixture thereof.

5. The composition for use according to any one of the preceding claims, wherein there are also present natural or synthetic excipients and/or emulsifiers selected from petrolatum jelly, glycerine, paraffin, cetearyl glucose, beeswax or rice wax, soya lecithin, sugar esters, glyceryl stearate, olive oil derivatives or a mixture thereof.

6. The composition for use according to any one of the preceding claims, wherein the composition comprises from 60 to 95 parts by weight of electrolyzed water, from 0 to 10 parts by weight of synthetic or natural healing agent and from 1 to 10 parts by weight of an excipient and/or emulsifiers.

7. The composition for use according to any one of claims 1 to 5 wherein, the composition comprises from 60 to 95 parts by weight of an excipient and/or emulsifiers, from 0 to 10 parts by weight of synthetic or natural healing agent and from 10 to 30 parts by weight of electrolyzed water.

8. The composition according to any one of the preceding claims for use as a wound-healing medicament in the treatment of skin conditions generating wounds or defects in healing, such as bedsores, erysipelas, open wounds, varicose ulcers.

9. The skin application mask comprising a composition according to one of claims 1 to 8.
